# EUROPEAN PATENT SPECIFICATION

(11) **EP 0 544 716 B1**
(45) Date of publication and mention of the grant of the patent: **18.12.2002**
(21) Application number: 91914455.0
(22) Date of filing: 23.07.1991
(51) Int. Cl.: C12N 15/11, A61K 31/70

(54) **OLIGONUCLEOTIDE MODULATION OF CELL ADHESION**
BEEINFLUSSUNG DER ZELLADHÄSION DURCH OLIGONUKLEOTIDE
MODULATION PAR DES OLIGONUCLEOTIDES DE L'ADHERENCE CELLULAIRE

(30) Priority: 14.08.1990 US 567286
(43) Date of publication of application: 09.06.1993
(73) Proprietor: ISIS PHARMACEUTICALS, INC., Carlsbad, CA 92008 (US)
(72) Inventor: BENNETT, Clarence, Frank, Carlsbad, CA 92008 (US); MIRABELLI, Christopher, K., Encinitas, CA 92024 (US)
(74) Representative: Hallybone, Huw George
(86) International application number: US9105209
(87) International publication number: WO92003139

(56) References cited:
- WO-A-89/06702
- WO-A-90/13300
- JOURNAL OF THE NATIONAL CANCER INSTITUTE vol. 81, no. 20 , 18 October 1989 , BETHESDA, MD, US pages 39 - 44 ROTHENBERG, M. ET AL. 'Oligodeoxynucleotides as anti-sense inhibitors of gene expression: therapeutic implications'
- LYMPHOKINE RESEARCH vol. 9, no. 1 , 1990 pages 35 - 42 MANSON, J. ET AL. 'Modulation of interleukin 1beta gene expression using antisense phosphorothioate oligonucleotides'
- CLINICAL RESEARCH vol. 39, no. 2 , 1991 page 195A HEALD, P. ET AL. 'Antisense oligonucleotide inhibition of intracellular adhesion molecule-1 (ICAM-1) induction in normal human keratinocytes' & Joint meeting of the Association of American Physicians, the American Society for Clinical Investigation, and the American Federation for Clinical Research;
- JOURNAL OF BIOLOGICAL CHEMISTRY vol. 266, no. 27 , 25 September 1991 , BALTIMORE, MD US pages 18162 - 18171 CHIANG, M.-Y. ET AL. 'Antisense oligonucleotides inhibit intercellular adhesion molecule 1 expression by two distinct mechanisms'
- Pharmaceutical Research, Volume 5, No. 9, issued 1988, Zon, "Oligonucleotide Analogues as Potential Chemotherapeutic Agents", pages 539-549, see entire document.
- Cell, Volume 52, issued 25 March 1988, D.E. STAUTON et al., "Primary Structure of ICAM-1 demonstrates Interaction between members of the Immunoglobulin and Integrin Supergene Families", pages 925-933, see entire documents.
- Science, Volume 243, issued 03 March 1989, M.P. BEVILACQUA et al., "Endothelial Leukocyte Adhesion Molecule 1: An induc ible receptor for neutrophils related to Complement Regulatory Proteins and Lectins", pages 1160-1165, see entire documents.
- Cell, Volume 9, issued December 1989, L. OSBORN et al., "Direct Expression coloning of Vascular cell adhesion Molecule a cyto kine-induced Endothelical Protein that binds to Lymphocytes", pages 1203-1211, see entire article.

## Description

### FIELD OF THE INVENTION

This invention relates to diagnostics, research reagents, and therapies for disease states which respond to modulation of the synthesis or metabolism of cell adhesion molecules. In particular, this invention relates to antisense oligonucleotide interactions with certain messenger ribonucleic acids (mRNAs) or DNAs involved in the synthesis of proteins that regulate adhesion of white blood cells to other white blood cells and to other cell types. Antisense oligonucleotides designed to hybridize to the mRNA encoding intercellular adhesion molecule-1 (ICAM-1) are provided. These oligonucleotides have been found to lead to the modulation of the activity of the RNA or.DNA, and thus to the modulation of the synthesis and metabolism of specific cell adhesion molecules. Palliation and therapeutic effect result.

### BACKGROUND OF THE INVENTION

Inflammation is a localized protective response elicited by tissues in response to injury, infection, or tissue destruction resulting in the destruction of the infectious or injurious agent and isolation of the injured tissue. A typical inflammatory response proceeds as follows: recognition of an antigen as foreign or recognition of tissue damage, synthesis and release of soluble inflammatory mediators, recruitment of inflammatory cells to the site of infection or tissue damage, destruction and removal of the invading organism or damaged tissue, and deactivation of the system once the invading organism or damage has been resolved. In many human diseases with an inflammatory component, the normal, homeostatic mechanisms which attenuate the inflammatory responses are defective, resulting in damage and destruction of normal tissue.

Cell-cell interactions are involved in the activation of the immune response at each of the stages described above. One of the earliest detectable events in a normal inflammatory response is adhesion of leukocytes to the vascular endothelium, followed by migration of leukocytes out of the vasculature to the site of infection or injury. The adhesion of these leukocytes, or white blood cells, to vascular endothelium is an obligate step in the migration out of the vasculature. Harlan, J.M., *Blood* 65: 513-525 (1985). In general, the first inflammatory cells to appear at the site of inflammation are neutrophils followed by monocytes, and lymphocytes. Cell-cell interactions are also critical for propagation of both B-lymphocytes and T-lymphocytes resulting in enhanced humoral and cellular immune responses, respectively.

The adhesion of white blood cells to vascular endothelium and other cell types is mediated by interactions between specific proteins, termed "adhesion molecules," located on the plasma membrane of both white blood cells and vascular endothelium. The interaction between adhesion molecules is similar to classical receptor ligand interactions with the exception that the ligand is fixed to the surface of a cell instead of being soluble. The identification of patients with a genetic defect in leukocyte adhesion has enabled investigators to identify a family of proteins responsible for adherence of white blood cells. Leukocyte adhesion deficiency (LAD) is a rare autosomal trait characterized by recurrent bacterial infections and impaired pus formation and wound healing. The defect was shown to occur in the common B-subunit of three heterodimeric glycoproteins, Mac-1, LFA-1, and p150,95, normally expressed on the outer cell membrane of white blood cells. Anderson and Springer, *Ann. Rev*. *Med.* 38:175-194 (1987). Patients suffering from LAD exhibit a defect in a wide spectrum of adherence-dependent functions of granulocytes, monocytes, and lymphocytes. Two ligands for LFA-1 have been identified, intercellular adhesion molecules 1 and 2 (ICAM-1 and ICAM-2). Both Mac-1 and p150, 95 bind complement fragment C3bi and perhaps other unidentified ligands.

Other adhesion molecules have been identified which are involved in the adherence of white blood cells to vascular endothelium and subsequent migration out of the vasculature. These include endothelial leukocyte adhesion molecule-1 (ELAM-1), vascular cell adhesion molecule-1 (VCAM-1) and granule membrane protein-140 (GMP-140) and their respective receptors. The adherence of white blood cells to vascular endothelium appears to be mediated in part if not *in toto* by the five cell adhesion molecules ICAM-1, ICAM-2, ELAM-1, VCAM-1 and GMP-140. Dustin and Springer, *J. Cell Biol.,* 107:321-331 (1987). Expression on the cell surface of ICAM-1, ELAM-1, VCAM-1 and GMP-140 adhesion molecules is induced by inflammatory stimuli. In contrast, expression of ICAM-2 appears to be constitutive and not sensitive to induction by cytokines. In general, GMP-140 is induced by autocoids such as histamine, leukotriene B₄, platelet activating factor, and thrombin. Maximal expression on endothelial cells is observed 30 minutes to 1 hour after stimulation and returns to baseline within 2 to 3 hours. The expression of ELAM-1 and VCAM-1 on endothelial cells is induced by cytokines such as interleukin-1β and tumor necrosis factor, but not gamma-interferon. Maximal expression of ELAM-1 on the surface of endothelial cells occurs 4 to 6 hours after stimulation and returns to baseline by 16 hours. ELAM-1 expression is dependent on new mRNA and protein synthesis. Elevated VCAM-1 expression is detectable 2 hours following treatment with tumor necrosis factor, is maximal 8 hours following stimulation, and remains elevated for at least 48 hours following stimulation. Rice and Bevilacqua, *Science,* 246:1303-1306 (1989). ICAM-1 expression on endothelial cells is induced by cytokines interleukin-1 tumor necrosis factor and gamma-interferon. Maximal expression of ICAM-1 follows that of ELAM-1 occurring 8 to 10 hours after stimulation and remains elevated for at least 48 hours.

GMP-140 and ELAM-1 are primarily involved in the adhesion of neutrophils to vascular endothelial cells. VCAM-1 primarily binds T and B lymphocytes. In addition, VCAM-1 may play a role in the metastasis of melanoma, and possibly other cancers. ICAM-1 plays a role in adhesion of neutrophils to vascular endothelium, as well as adhesion of monocytes and lymphocytes to vascular endothelium, tissue fibroblasts and epidermal keratinocytes. ICAM-1 also plays a role in T-cell recognition of antigen presenting cell, lysis of target cells by natural killer cells, lymphocyte activation and proliferation, and maturation of T cells in the thymus. In addition, recent data have demonstrated that ICAM-1 is the cellular receptor for the major serotype of rhinovirus, which account for greater than 50% of common colds. Staunton et al., *Cell,* 56: 849-853 (1989); Greve et al., *Cell,* 56: 839-847 (1989).

Expression of ICAM-1 has been associated with a variety of inflammatory skin disorders such as allergic contact dermatitis, fixed drug eruption, lichen planus, and psoriasis; Ho et al., *J. Am. Acad*. *Dermatol*., 22: 64-68 (1990); Griffiths and Nickoloff, *Am. J. Pathology,* 135: 1045-1053 (1989); Lisby et al., *Br*. *J. Dermatol*., 120:479-484 (1989); Shiohara et al., *Arch. Dermatol*., 125: 1371-1376 (1989). In addition, ICAM-1 expression has been detected in the synovium of patients with rheumatoid arthritis; Hale et al., *Arth*. *Rheum.*, 32: 22-30 (1989), pancreatic B-cells in diabetes; Campbell et al., *Proc. Natl. Acad. Sci. U.S.A*., 86:4282-4286 (1989); thyroid follicular cells in patients with Graves' disease; Weetman et al., *J. Endocrinol.,* 122: 185-191 (1989); and with renal and liver allograft rejection; Faull and Russ, *Transplantation,* 48: 226-230 (1989); Adams et al., *Lancet*, 1122-1125 (1989).

It is has been hoped that inhibitors of ICAM-1, VCAM-1 and ELAM-1 expression would provide a novel therapeutic class of anti-inflammatory agents with activity towards a variety of inflammatory diseases or diseases with an inflammatory component such as asthma, rheumatoid arthritis, allograft rejections, various dermatological conditions, and psoriasis. In addition, inhibitors of ICAM-1, VCAM-1, and ELAM-1 may also be effective in the treatment of colds due to rhinovirus infection, AIDS, and some cancers and their metastasis. To date, there are no known therapeutic agents which effectively prevent the expression of the cellular adhesion molecules ELAM-1, VCAM-1 and ICAM-1. The use of neutralizing monoclonal antibodies against ICAM-1 in animal models provide evidence that such inhibitors if identified would have therapeutic benefit for asthma; Wegner et al., *Science,* 247:456-459 (1990) and renal allografts; Cosimi et al., *J. Immunol.,* 144:4604-4612 (1990). The use of a soluble form of ICAM-1 molecule was also effective in preventing rhinovirus infection of cells in culture. Marlin et al., *Nature*, 344:70-72 (1990).

Current agents which affect intercellular adhesion molecules include synthetic peptides, monoclonal antibodies, and soluble forms of the adhesion molecules. To date, synthetic peptides which block the interactions with ICAM-1, VCAM-1 or ELAM-1 have not been identified. Monoclonal antibodies may prove to be useful for the treatment of acute inflammatory response due to expression of ICAM-1, VCAM-1 and ELAM-1. However, with chronic treatment, the host animal develops antibodies against the monoclonal antibodies thereby limiting their usefulness. In addition, monoclonal antibodies are large proteins which may have difficulty in gaining access to the inflammatory site. Soluble forms of the cell adhesion molecules suffer from many of the same limitations as monoclonal antibodies in addition to the expense of their production. Thus, there is a long felt need for molecules which effectively inhibit intercellular adhesion molecules. Antisense oligonucleotides avoid many of the pitfalls of current agents used to block the effects of ICAM-1, VCAM-1 and ELAM-1.

WO90/13300 (Biogen Inc) discloses DNA sequences encoding Endothelial Adhesion Molecules (ELAMs), including ELAM-1 and VCAM-1 and VCAM-1b. A number of uses for these DNA sequences are provided, including (1) production of monoclonal antibody preparations that are reactive for these molecules which may be used as therapeutic agents to inhibit leukocyte binding to endothelial cells; (2) production of ELAM peptides to bind to the ELAM ligand on leukocytes which, in turn, may bind to ELAM on endothelial cells, inhibiting leukocyte binding to endothelial cells; (3) use of molecules binding to ELAMS (such as anti-ELAM antibodies, or markers such as the ligand or fragments of it) to detect inflammation; (4) use of ELAM and ELAM ligand DNA sequences to produce nucleic acid molecules that intervene in ELAM or FIRM ligand expression at the translational level using antisense nucleic acid and ribozymes to block translation of a specific mRNA either by masking mRNA with antisense nucleic acid or cleaving it with a ribozyme. It is disclosed that coding regions are the targets of choice. For VCAM-1, AUG is believed to be most likely; a 15-mer hybridizing to the AUG site is specifically disclosed in Example 17.

### OBJECTS OF THE INVENTION

It is a principle object of the invention to provide therapies for diseases with an immunological component, allografts, cancers and metastasis, colds, and AIDS through perturbation in the synthesis and expression of inflammatory cell adhesion molecules.

It is a further object of the invention to provide antisense oligonucleotides or oligonucleotide analogs which are capable of inhibiting the function of nucleic acids encoding intercellular adhesion proteins.

Yet another object is to provide means for diagnosis of dysfunctions of intercellular adhesion.

These and other objects of this invention will become apparent from a review of the instant specification.

### BRIEF DESCRIPTION OF THE DRAWINGS

FIGURE 1 is the mRNA sequence of human intercellular adhesion molecule-1 (ICAM-1).
FIGURE 2 is a graphical representation of the induction of ICAM-1 expression on the cell surface of various human cell lines by interleukin-1 and tumor necrosis factor.
FIGURE 3 is a graphical representation of the effects of selected antisense oligonucleotides on ICAM-1 expression on human umbilical vein endothelial cells.
FIGURE 4A and 4B are a graphical representation of the effects of an antisense oligonucleotide on the expression of ICAM-1 in human umbilical vein endothelial cells stimulated with tumor necrosis factor and interleukin-1.
FIGURE 5 is a graphical representation of the effect of antisense oligonucleotides on ICAM-1 mediated adhesion of DMSO differentiated HL-60 cells to control and tumor necrosis factor treated human umbilical vein endothelial cells.
FIGURE 6 is a graphical representation of the effects of selected antisense oligonucleotides on ICAM-1 expression in A549 human lung carcinoma cells.
FIGURE 7 is a graphical representation of the effects of selected antisense oligonucleotides on ICAM-1 expression in primary human keratinocytes.
FIGURE 8 is a graphical representation of the relationship between oligonucleotide chain length, Tm· and effect on inhibition of ICAM-1 expression.
FIGURE 9 is a graphical representation of the effect of selected antisense oligonucleotides on ICAM-1 mediated adhesion of DMSO differentiated HL-60 cells to control and tumor necrosis factor treated human umbilical vein endothelial cells.

### SUMMARY OF THE INVENTION

In accordance with the present invention, oligonucleotides and oligonucleotide analogs are provided which specifically hybridize with nucleic acids encoding intercellular adhesion molecule-1 (ICAM-1). The oligonucleotides and oligonucleotide analogs are designed to. bind either directly to mRNA or to a selected DNA portion forming a triple stranded structure, thereby modulating the amount of mRNA made from the gene.

The former relationship is commonly denoted as "antisense." The oligonucleotides and oligonucleotide analogs are able to inhibit the function of RNA or DNA, either its translation into protein, its translocation into the cytoplasm, or any other activity necessary to its overall biological function. The failure of the RNA or DNA to perform all or part of its function results in failure of a portion of the genome controlling cell adhesion molecules to be properly expressed, thus modulating said metabolism.

It is preferred to target specific genes for antisense attack. It has been discovered that the genes coding for ICAM-1, VCAM-1 and ELAM-1 are particularly useful for this approach. Inhibition of ICAM-1 expression is expected to be useful for the treatment of inflammatory diseases, diseases with an inflammatory component, allografts, cancers and their metastasis, and viral infections.

Methods of modulating cell adhesion comprising contacting the animal with an oligonucleotide or oligonucleotide analog hybridizable with nucleic acids encoding a protein capable of modulating cell adhesion are provided. Oligonucleotides or oligonucleotide analogs hybridizable with an RNA or DNA encoding ICAM-1 are preferred. Methods for diagnosis are also a part of this invention.

### DETAILED DESCRIPTION OF PREFERRED EMBODIMENTS

Antisense oligonucleotides hold great promise as therapeutic agents for the treatment of many human diseases. Conceptually, it is much easier to design compounds which interact with a primary structure of a molecule such as an RNA molecule by base pairing than it is to design a molecule to interact with the active site of an enzyme or ligand binding site of a receptor. Oligonucleotides specifically bind to the complementary sequence of either pre-mRNA or mature mRNA, as defined by Watson-Crick base pairing, inhibiting the flow of genetic information from DNA to protein. The properties of antisense oligonucleotides which make them specific for their target sequence also make them extraordinarily versatile. Because antisense oligonucleotides are long chains of four monomeric units they may be readily synthesized for any target RNA sequence. Numerous recent studies have documented the utility of antisense oligonucleotides as biochemical tools for studying target proteins. Rothenberg et al., *J. Natl*. *Cancer Inst.,* 81:1539-1544 (1989); Zon, *G. Pharmaceutical Res.,* 5:539-549). Because of recent advances in oligonucleotide chemistry, synthesis of nuclease resistant oligonucleotides, and oligonucleotide analogs which exhibit enhanced cell uptake, it is now possible to consider the use of antisense oligonucleotides as a novel form of therapeutics.

Antisense oligonucleotides offer an ideal solution to the problems encountered in prior art approaches. They can be designed to selectively inhibit a given isoenzyme, they inhibit the production of the enzyme, and they avoid non-specific mechanisms such as free radical scavenging or binding to multiple receptors. A complete understanding of enzyme mechanisms or receptor-ligand interactions is not needed to design specific inhibitors.

### DESCRIPTION OF TARGETS

The acute infiltration of neutrophils into the site of inflammation appears to be due to increased expression of GMP-140, ELAM-1 and ICAM-1 on the surface of endothelial cells. The appearance of lymphocytes and monocytes during the later stages of an inflammatory reaction appear to be mediated by VCAM-1 and ICAM-1. ELAM-1 and GMP-140 are transiently expressed on vascular endothelial cells, while VCAM-1 and ICAM-1 are chronically expressed.

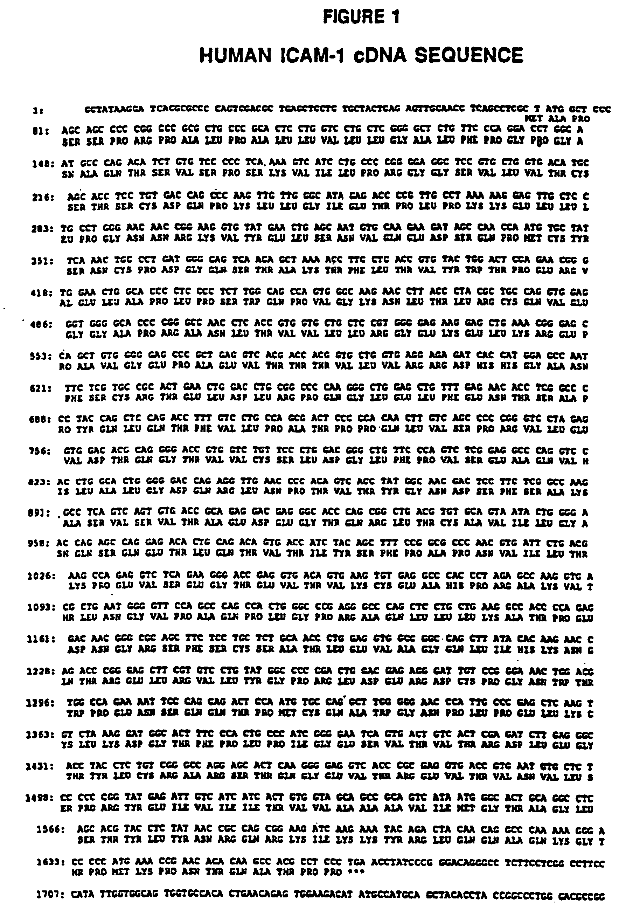

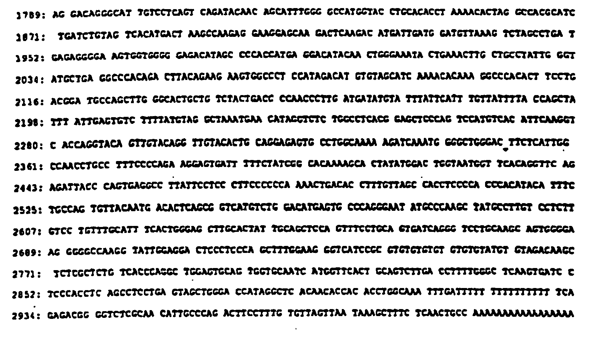

Human ICAM-1 is encoded by a 3.3-kb mRNA resulting in the synthesis of a 55,219 dalton protein (Figure 1). ICAM-1 is heavily glycosylated through N-linked glycosylation sites. The mature protein has an apparent molecular mass of 90 kDa as determined by SDS-polyacrylamide gel electrophoresis. Staunton et al., *Cell,* 52:925-933. ICAM-1 is a member of the immunoglobulin supergene family, containing 5 immunoglobulin-like domains at the amino terminus, followed by a transmembrane domain and a cytoplasmic domain. The primary binding site for LFA-1 and rhinovirus are found in the first immunoglobulin-like domain. However, the binding sites appear to be distinct. Staunton et al., *Cell*, 61:243-354 (1990). Recent electron micrographic studies demonstrate that ICAM-1 is a bent rod 18.7 nm in length and 2 to 3 nm in diameter. Staunton et al., *Cell,* 61:243-254 (1990).

ICAM-1 exhibits a broad tissue and cell distribution, and may be found on white blood cells, endothelial cells, fibroblast, keratinocytes and other epithelial cells. The expression of ICAM-1 can be regulated on vascular endothelial cells, fibroblasts, keratinocytes, astrocytes and several cell lines by treatment with bacterial lipopolysaccharide and cytokines such as interleukin-1, tumor necrosis factor, gamma-interferon, and lymphotoxin. *See, e.g.*, Frohman, et al., *J. Neuroimmunol*., 23:117-124 (1989). The molecular mechanism for increased expression of ICAM-1 following cytokine treatment has not been determined.

For therapeutics, an animal suspected of having a disease which can be treated by decreasing the expression of ICAM-1 is treated by administering oligonucleotides or oligonucleotide analogs in accordance with this invention. Persons of ordinary skill can easily determine optimum dosages, dosing methodologies and repetition rates. Such treatment is generally continued until either a cure is effected or a diminution in the disease state is achieved. Long term treatment is likely for some diseases.

The present invention employs oligonucleotides and oligonucleotide analogs for use in antisense inhibition of the function of RNA and DNA corresponding to proteins capable of modulating inflammatory cell adhesion. In the context of this invention, the term "oligonucleotide" refers to a polynucleotide formed from naturally occurring bases and furanosyl groups joined by native phosphodiester bonds. This term effectively refers to naturally occurring species or synthetic species formed from naturally occurring subunits or their close homologs.

"Oligonucleotide analog," as that term is used in connection with this invention, refers to moieties which function similarly to oligonucleotides but which have non-naturally occurring portions. Thus, oligonucleotide analogs may have altered sugar moieties or inter-sugar linkages. Exemplary among these are the phosphorothioate and other sulfur-containing species which are known for use in the art. In accordance with some preferred embodiments, at least some of the phosphodiester bonds of the oligonucleotide have been substituted with a structure which functions to enhance the ability of the compositions to penetrate into the region of cells where the RNA or DNA whose activity to be modulated is located. It is preferred that such substitutions comprise phosphorothioate bonds, methyl phosphonate bonds, or short chain alkyl or cycloalkyl structures. In accordance with other preferred embodiments, the phosphodiester bonds are substituted with other structures which are, at once, substantially nonionic and non-chiral, or with structures which are chiral and enantiomerically specific. Persons of ordinary skill in the art will be able to select other linkages for use in practice of the invention.

Oligonucleotide analogs may also include species which include at least some modified base forms. Thus, purines and pyrimidines other than those normally found in nature may be so employed. Similarly, modifications on the furanosyl portions of the nucleotide subunits may also occur as long as the essential tenets of this invention are adhered to. Examples of such modifications are 2'-O-alkyl- and 2'-halogen-substituted nucleotides.

Such analogs are best described as being functionally interchangeable with natural oligonucleotides (or oligonucleotides synthesized along natural lines), but which have one or more differences from natural structure. All such analogs are comprehended by this invention so long as they function effectively to hybridize with RNA and DNA deriving from a gene corresponding to one of the proteins capable of modulating intercellular adhesion. The oligonucleotides and oligonucleotide analogs in accordance with this invention preferably comprise from about 3 to about 50 subunits. It is more preferred that such oligonucleotides and oligonucleotide analogs comprise from about 8 to 25 subunits, and still more preferred to have from about 12 to 22 subunits. As will be appreciated, a subunit is a base-sugar combination suitably bound to adjacent subunits through phosphodiester or other bonds.

The oligonucleotides and oligonucleotide analogs used in accordance with this invention may be conveniently and routinely made through the well-known technique of solid phase synthesis. Equipment for such synthesis is sold by several vendors including Applied Biosystems. Any other means for such synthesis may also be employed, however the actual synthesis of the oligonucleotides is well within the talents of the routineer. It is also well known to use similar techniques to prepare other oligonucleotide analogs such as the phosphorothioates and alkylated derivatives.

In accordance with this invention, persons of ordinary skill in the art will understand that messenger RNA identified by the open reading frames (ORFs) of the DNA from which they are transcribed includes not only the information from the ORFs of the DNA, but also associated ribonucleotides which form regions known to such persons as the 5'-untranslated region, the 3'- untranslated region, and intervening sequence ribonucleotides. Thus, oligonucleotides and oligonucleotide analogs may be formulated in accordance with this invention which are targeted wholly or in part to these associated ribonucleotides as well as to the informational ribonucleotides. In preferred embodiments, the oligonucleotide or oligonucleotide analog is specifically hybridizable with a transcription initiation site, a translation initiation site, an intervening sequence and sequences in the 3'-untranslated region.

In accordance with this invention, the oligonucleotide is specifically hybridizable with portions of nucleic acids encoding a protein involved in the adhesion of white blood cells either to other white blood cells or other cell types. In preferred embodiments, said protein is intercellular adhesion molecule-1. Oligonucleotides or analogs comprising the corresponding sequence, or part thereof, are useful in the invention.

Several preferred embodiments of this invention are exemplified in accordance with the following nonlimiting examples. The target mRNA species for modulation relates to intercellular adhesion molecule-1. Persons of ordinary skill in the art will appreciate that the present invention is not so limited, however, and that it is generally applicable. The inhibition or modulation of production of ICAM-1 is expected to have significant therapeutic benefits in the treatment of disease. In order to assess the effectiveness of the compositions, an assay or series of assays is required.

### EXAMPLES

### EXAMPLE 1

Expression of ICAM-1 on the surface of cells can be quantitated using specific monoclonal antibodies in an ELISA. Cells are grown to confluence in 96 well microtiter plates. The cells are stimulated with either interleukin-1 or tumor necrosis factor for 8 to 24 hours to quantitate ICAM-1. Following the appropriate incubation time with the cytokine, the cells are gently washed three times with a buffered isotonic solution containing calcium and magnesium such as Dulbecco's phosphate buffered saline (D-PBS). The cells are then directly fixed on the microtiter plate with 1 to 2% paraformaldehyde diluted in D-PBS for 20 minutes at 25° C. The cells are washed again with D-PBS three times. Nonspecific binding sites on the microtiter plate are blocked with 2% bovine serum albumin in D-PBS for 1 hour at 37° C. Cells are incubated with the appropriate monoclonal antibody diluted in blocking solution for 1 hour at 37°C. Unbound antibody is removed by washing the cells three times with D-PBS. Antibody bound to the cells is detected by incubation with a 1:1000 dilution of biotinylated goat anti-mouse IgG (Bethesda Research Laboratories, Gaithersberg, MD) in blocking solution for 1 hour at 37°C. Cells are washed three times with D-PBS and then incubated with a 1:1000 dilution of streptavidin conjugated to β-galactosidase (Bethesda Research Laboratories) for 1 hour at 37°C. The cells are washed three times with D-PBS for 5 minutes each. The amount of β-galactosidase bound to the specific monoclonal antibody is determined by developing the plate in a solution of 3.3 mM chlorophenolred-β-D-galactopyranoside, 50 mM sodium phosphate, 1.5 mM MgCl₂; pH=7.2 for 2 to 15 minutes at 37°C. The concentration of the product is determined by measuring the absorbance at 575 nm in an ELISA microtiter plate reader.

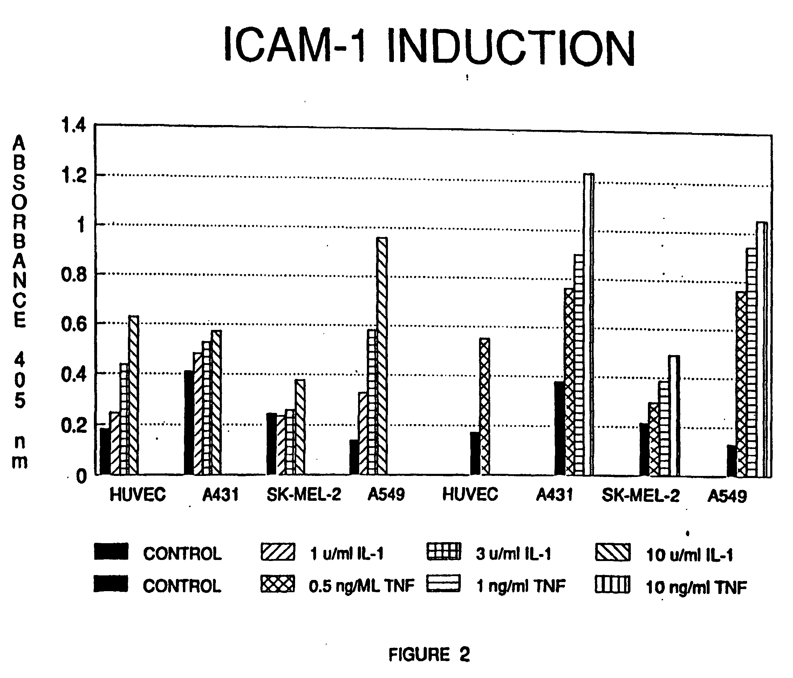

An example of the induction of ICAM-1 observed following stimulation with either interleukin-1β or tumour necrosis factor α in several human cell lines is shown in Figure 2. Cells were stimulated with increasing concentrations of interleukin-1 or tumour necrosis factor for 15 hours and processed as described above. ICAM-1 expression was determined by incubation with a 1:1000 dilution of the monoclonal antibody 84H10 (Amac Inc., Westbrook, ME). The cell lines used were passage 4 human umbilical vein endothelial cells (HUVEC), a human epidermal carcinoma cell line (A431), a human melanoma cell line (SK-MEL-2) and a human lung carcinoma cell line (A549). ICAM-1 was induced on all the cell lines, however, tumor necrosis factor was more effective than interleukin-1 in induction of ICAM-1 expression on the cell surface (Figure 2.).

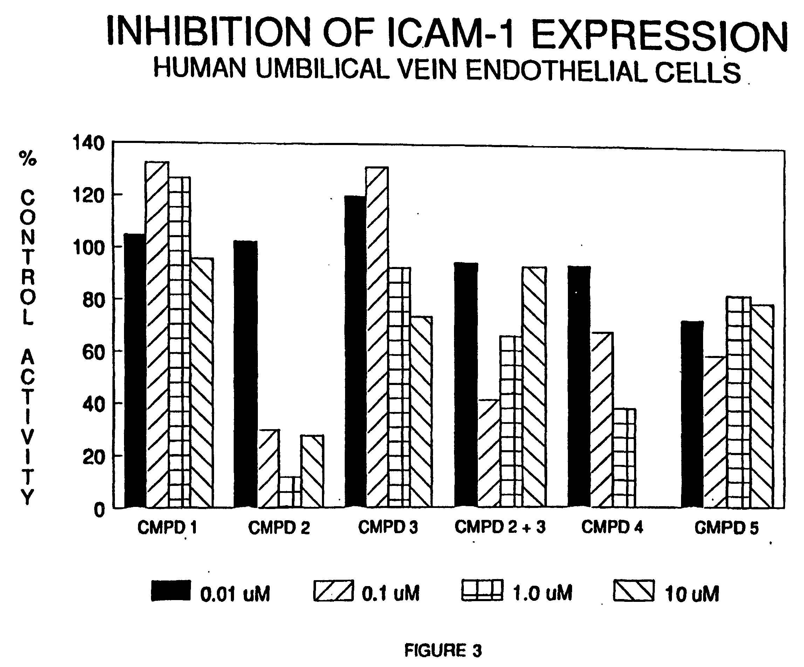

Screening antisense oligonucleotides for inhibition of ICAM-1 expression is performed as described above with the exception of pretreatment of cells with the oligonucleotides prior to challenge with the cytokines. An example of antisense oligonucleotide inhibition of ICAM-1 expression is shown in Figure 3. Human umbilical vein endothelial cells (HUVEC) were treated with increasing concentration of oligonucleotide diluted in Opti MEM (GIBCO, Grand Island, NY) containing 16 µM N-[1-(2,3-dioleyloxy) propyl]-N,N,N-trimethylammonium chloride (DOTMA) for 4 hours at 37°C to enhance uptake of the oligonucleotides. The medium was removed and replaced with endothelial growth medium (EGM-UV; Clonetics, San Diego, CA) containing the indicated concentration of oligonucleotide for an additional 4 hours. Interleukin- 1β was added to the cells at a concentration of 5 units/ml and incubated for 14 hours at 37°C. The cells were quantitated for ICAM-1 expression using a 1:1000 dilution of the monoclonal antibody 84H10 as described above. The oligonucleotides used were:
**COMPOUND 1 -** (ISIS 1558) a phosphodiester oligonucleotide designed to hybridize with position 64-80 of the mRNA covering the AUG initiation of translation codon having the sequence
**COMPOUND 2-** (ISIS 1570) a phosphorothioate containing oligonucleotide corresponding to the same sequence as COMPOUND 1.
**COMPOUND 3 -** a phosphorothioate oligonucleotide complementary to COMPOUND 1 and COMPOUND 2 exhibiting the sequence
**COMPOUND 4 -** (ISIS 1572) a phosphorothioate containing oligonucleotide designed to hybridize to positions 2190-2210 of the mRNA in the 3'untranslated region containing the sequence
**COMPOUND 5 -** (ISIS 1821) a phosphorothioate containing oligonucleotide design to hybridize to human 5-lipoxygenase mRNA used as a control containing the sequence

The phosphodiester oligonucleotide targeting the AUG initiation of translation region of the human ICAM-1 mRNA (COMPOUND 1) failed to inhibit expression of ICAM-1, however, the corresponding phosphorothioate containing oligonucleotide (COMPOUND 2) inhibited ICAM-1 expression by 70% at a concentration of 0.1 µM and 90% at 1 µM concentration (Figure 3). The increased potency of the phosphorothioate oligonucleotide over the phosphodiester is probably due to increased stability. The sense strand to COMPOUND 2, COMPOUND 3, modestly inhibited ICAM-1 expression at 10 µM. If COMPOUND 2 was prehybridized to COMPOUND 3 prior to addition to the cells, the effects of COMPOUND 2 on ICAM-1 expression were attenuated suggesting that the activity of COMPOUND 2 was due to antisense oligonucleotide effect, requiring hybridization to the mRNA. The antisense oligonucleotide directed against 3' untranslated sequences (COMPOUND 4) inhibited ICAM-1 expression 62% at a concentration of 1 µM (Figure 3). The control oligonucleotide, targeting human 5-lipoxygenase (COMPOUND 5) reduced ICAM-1 expression by 20%. These data demonstrate that oligonucleotides are capable of inhibiting ICAM-1 expression on human umbilical vein endothelial cells and suggest that the inhibition of ICAM-1 expression is due to an antisense activity.

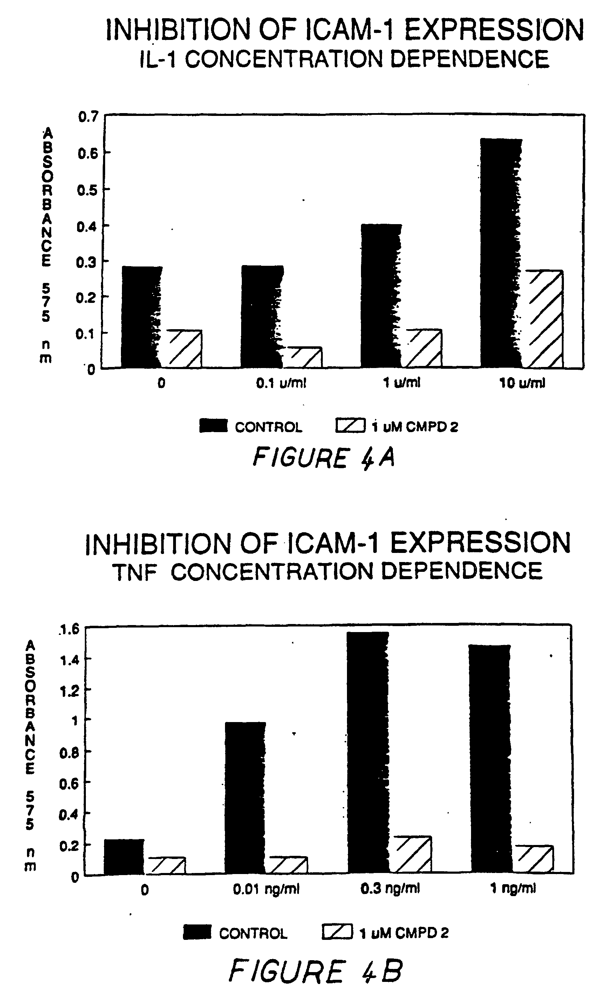

The antisense oligonucleotide COMPOUND 2 at a concentration of 1 µM inhibits expression of ICAM-1 on human umbilical vein endothelial cells stimulated with increasing concentrations of tumor necrosis factor and interleukin-1 (Figure 4). These data demonstrate that the effects of COMPOUND 2 are not specific for interleukin-1 stimulation of cells.

### EXAMPLE 2

A second cellular assay which can be used to demonstrate the effects of antisense oligonucleotides on ICAM-1 expression is a cell adherence assay. Target cells are grown as a monolayer in a multiwell plate, treated with oligonucleotide followed by cytokine. The adhering cells are then added to the monolayer cells and incubated for 30 to 60 minutes at 37°C and washed to remove nonadhering cells. Cells adhering to the monolayer may be determined either by directly counting the adhering cells or prelabeling the cells with a radioisotope such as ⁵¹Cr and quantitating the radioactivity associated with the monolayer as described. Dustin and Springer, *J. Cell Biol.,* 107:321-331 (1988).

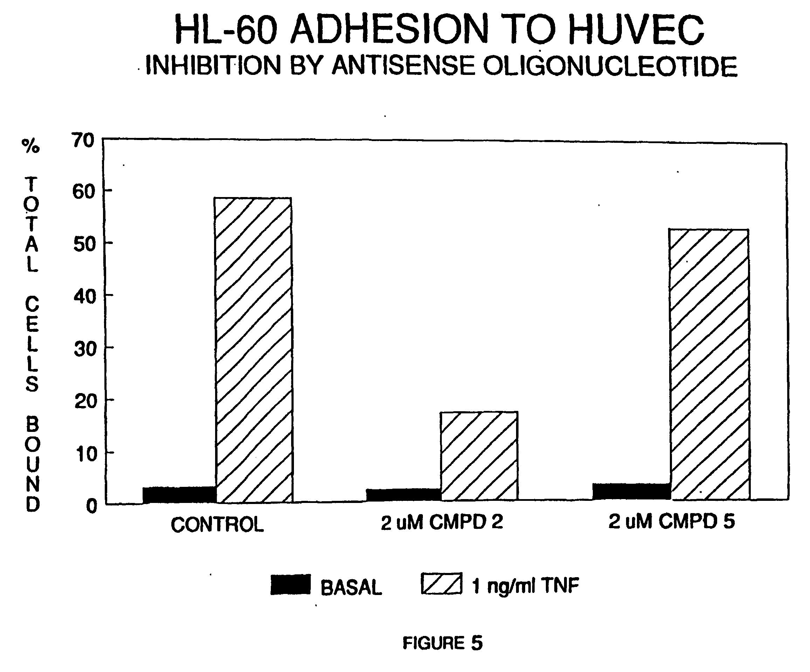

An example of the effects of antisense oligonucleotides targeting ICAM-1 mRNA on the adherence of DMSO differentiated HL-60 cells to tumor necrosis factor treated human umbilical vein endothelial cells is shown in Figure 5. Human umbilical vein endothelial cells were grown to 80% confluence in 12 well plates. The cells were treated with 2 µM oligonucleotide diluted in Opti-MEM containing 8 µM DOTMA for 4 hours at 37°C. The medium was removed and replaced with fresh endothelial cell growth medium (EGM-UV) containing 2 µM of the indicated oligonucleotide and incubated 4 hours at 37°C. Tumor necrosis factor, 1 ng/ml, was added to cells as indicated and cells incubated for an additional 19 hours. The cells were washed once with EGM-UV and 1.6 x 10⁵ HL-60 cells differentiated for 4 days with 1.3% DMSO added. The cells were allowed to attach for 1 hour at 37°C and gently washed 4 times with Dulbecco's phosphate-buffered saline (D-PBS) warmed to 37°C. Adherent cells were detached from the monolayer by addition of 0.25 ml of cold (4°C) phosphate-buffered saline containing 5 mM EDTA and incubated on ice for 5 minutes. The number of cells removed by treatment with EDTA was determined by counting with a hemocytometer. Endothelial cells detached from the monolayer by EDTA treatment could easily be distinguished. from HL-60 cells by morphological differences.

In the absence of tumor necrosis factor, 3% of the HL-60 cells bound to the endothelial cells. Treatment of the endothelial cell monolayer with 1 ng/ml tumor necrosis factor increased the number of adhering cells to 59% of total cells added (Figure 5). Treatment with the antisense oligonucleotide COMPOUND 2 or the control oligonucleotide COMPOUND 5 did not change the number of cells adhering to the monolayer in the absence of tumor necrosis factor treatment (Figure 5). The antisense oligonucleotide, COMPOUND 2 reduced the number of adhering cells from 59% of total cells added to 17% of the total cells added (Figure 5). In contrast, the control oligonucleotide COMPOUND 5 did not significantly reduce the number of cells adhering to the tumor necrosis factor treated endothelial monolayer, i.e., 53% of total cells added for COMPOUND 5 treated cells versus 59% for control cells.

These data indicate that antisense oligonucleotides are capable of inhibiting ICAM-1 expression on endothelial cells and that inhibition of ICAM-1 expression correlates with a decrease in the adherence of a neutrophil-like cell to the endothelial monolayer in a sequence specific fashion. Because other molecules also mediate adherence of white blood cells to endothelial cells, such as ELAM-1, and VCAM-1 it is not expected that adherence would be completely blocked.

### EXAMPLE 3

### Synthesis and characterization of oligonucleotides and analogs:

Unmodified DNA oligonucleotides were synthesized on an automated DNA synthesizer (Applied Biosystems model 380B) using standard phosphoramidite chemistry with oxidation by iodine. β-cyanoethyldiisopropylphosphoramidites were purchased from Applied Biosystems (Foster City, CA). For phosphorothioate oligonucleotides, the standard oxidation bottle was replaced by a 0.2 M solution of 3H-1,2-benzodithiole-3-one 1,1-dioxide in acetonitrile for the stepwise thiation of the phosphite linkages. The thiation cycle wait step was increased to 68 seconds and was followed by the capping step.

2'-O-methyl phosphorothioate oligonucleotides were synthesized using 2'-O-methyl β-cyanoethyldiisopropylphosphoramidites (Chemgenes, Needham MA) and the standard cycle for unmodified oligonucleotides, except the wait step after pulse delivery of tetrazole and base was increased to 360 seconds. The 3'-base used to start the synthesis was a 2'-deoxyribonucleotide.

2'-fluoro phosphorothioate oligonucleotides were synthesized using 5'-dimethoxytrityl-3'-phosphoramidites and prepared as disclosed in U.S. patent application Serial No. 463,358, filed January 11, 1990, and 566,977, filed August 13, 1990, which are assigned to the same assignee as the instant application and which are incorporated by reference herein. The 2'-fluoro oligonucleotides were prepared using phosphoramidite chemistry and a slight modification of the standard DNA synthesis protocol: deprotection was effected using methanolic ammonia at room temperature.

After cleavage from the controlled pore glass column (Applied Biosystems) and deblocking in concentrated ammonium hydroxide at 55°C for 18 hours, the oligonucleotides were purified by precipitation twice out of 0.5 M NaCl with 2.5 volumes ethanol. Analytical gel electrophoresis was accomplished in 20% acrylamide, 8 M urea, 45 mM Tris-borate buffer, pH 7.0. Oligodeoxynucleotides and their phosphorothioate analogs were judged from electrophoresis to be greater than 80% full length material.

RNA oligonucleotide synthesis was performed on an ABI model 380B DNA synthesizer. The standard synthesis cycle was modified by increasing the wait step after the pulse delivery of tetrazole to 900 seconds. The bases were deprotected by incubation in methanolic ammonia overnight. Following base deprotections the oligonucleotides were dried *in vacuo.* The t-butyldimethylsilyl protecting the 2' hydroxyl was removed by incubating the oligonucleotide in 1 M tetrabutylammoniumfluoride in tetrahydrofuran overnight. The RNA oligonucleotides were further purified on C₁₈ Sep-Pak cartridges (Waters, Division of Millipore Corp., Milford MA) and ethanol precipitated.

The relative amounts of phosphorothioate and phosphodiester linkages obtained by this synthesis were periodically checked by ³¹P NMR spectroscopy. The spectra were obtained at ambient temperature using deuterium oxide or dimethyl sulfoxide-d₆ as solvent. Phosphorothioate samples typically contained less than one percent of phosphodiester linkages.

Secondary evaluation was performed with oligonucleotides purified by trityl-on HPLC on a PRP-1 column (Hamilton Co., Reno, Nevada) using a gradient of acetonitrile in 50 mM triethylammonium acetate, pH 7.0 (4% to 32% in 30 minutes, flow rate = 1.5 ml/min). Appropriate fractions were pooled, evaporated and treated with 5% acetic acid at ambient temperature for 15 minutes. The solution was extracted with an equal volume of ethyl acetate, neutralized with ammonium hydroxide, frozen and lyophilized. HPLC-purified oligonucleotides were not significantly different in potency from precipitated oligonucleotides, as judged by the ELISA assay for ICAM-1 expression.

### EXAMPLE 4

### Cell culture and treatment with oligonucleotides:

The human lung carcinoma cell line A549 was obtained from the American Type Culture Collection (Bethesda MD). Cells were grown in Dulbecco's Modified Eagle's Medium (Irvine Scientific, Irvine CA) containing 1 gm glucose/liter and 10% fetal calf serum (Irvine Scientific). Human umbilical vein endothelial cells (HUVEC) (Clonetics, San Diego CA) were cultured in EGM-UV medium (Clonetics). HUVEC were used between the second and sixth passages. Human epidermal carcinoma A431 cells were obtained from the American Type Culture Collection and cultured in DMEM with 4.5 g/l glucose. Primary human keratinocytes were obtained from Clonetics and grown in KGM (Keratinocyte growth medium, Clonetics).

Cells grown in 96-well plates were washed three times with Opti-MEM (GIBCO, Grand Island, NY) prewarmed to 37°C. 100 µl of Opti-MEM containing either 10 µg/ml N-[1-(2,3-dioleyloxy)propyl]-N,N,N-trimethylammonium chloride (DOTMA, Bethesda Research Labs, Bethesda MD) in the case of HUVEC cells or 20 µg/ml DOTMA in the case of A549 cells was added to each well. Oligonucleotides were sterilized by centrifugation through 0.2 µm Centrex cellulose acetate filters (Schleicher and Schuell, Keene, NH). Oligonucleotides were added as 20x stock solution to the wells and incubated for 4 hours at 37°C. Medium was removed and replaced with 150 µl of the appropriate growth medium containing the indicated concentration of oligonucleotide. Cells were incubated for an additional 3 to 4 hours at 37°C then stimulated with the appropriate cytokine for 14 to 16 hours, as indicated. ICAM-1 expression was determined as described in Example 1. The presence of DOTMA during the first 4 hours incubation with oligonucleotide increased the potency of the oligonucleotides at least 100-fold. This increase in potency correlated with an increase in cell uptake of the oligonucleotide.

### EXAMPLE 5

### ELISA screening of additional antisense oligonucleotides and oligonucleotide analogs for activity against ICAM-1 gene expression in Interleukin-1β-stimulated cells:

Antisense oligonucleotides were originally designed that would hybridize to five target sites on the human ICAM-1 mRNA. Oligonucleotides were synthesized in both phosphodiester (P=O; ISIS 1558, 1559, 1563, 1564 and 1565) and phosphorothioate (P=S; ISIS 1570, 1571, 1572, 1573, and 1574) forms. The oligonucleotides and analogs are shown in Table 1.

**TABLE 1**

| **ANTISENSE OLIGONUCLEOTIDES WHICH TARGET HUMAN ICAM-1** | | | |
|---|---|---|---|
| **ISIS NO.** | **SEO ID NO.** | **TARGET REGION** | **MODIFICATION** |
| 1558 | 1 | AUG Codon (64-81) | P=O |
| 1559 | 2 | 5'-Untranslated (32049) | P=O |
| 1563 | 3 | 3'-Untranslated (2190-3010) | P=O |
| 1564 | 4 | 3'-Untranslated (2849-2866) | P=O |
| 1565 | 5 | Coding Region (1378-1395) | P=O |
| 1570 | 1 | AUG Codon (64-81) | P=S |
| 1571 | 2 | 5'-Untranslated (32-49) | P=S |
| 1572 | 3 | 3'-Untranslated (2190-3010) | P=S |
| 1573 | 4 | 3'-Untranslated (2849-2866) | P=S |
| 1574 | 5 | Coding Region (1378-1395) | P=S |
| 1930 | 6 | 5'-Untranslated (1-20) | P=S |
| 1931 | 7 | AUG Codon (55-74) | P=S |
| 1932 | 8 | AUG Codon (72-91) | P=S |
| 1933 | 9 | Coding Region (111-130) | P=S |
| 1934 | 10 | Coding Region (351-370) | P=S |
| 1935 | 11 | Coding Region (889-908) | P=S |
| 1936 | 12 | Coding Region (1459-1468) | P=S |
| 1937 | 13 | Termination Codon (1651-1687) | P=S |
| 1938 | 14 | Termination Codon (1668-1687) | P=S |
| 1939 | 15 | 3'-Untranslated (1952-1971) | P=S |
| 1940 | 16 | 3'-Untranslated (2975-2994) | P=S |
| 2149 | 17 | AUG Codon (64-77) | P=S |
| 2163 | 18 | AUG Codon (64-75) | P=S |
| 2164 | 19 | AUG Codon (64-73) | P=S |
| 2165 | 20 | AUG Codon (66-80) | P=S |
| 2173 | 21 | AUG Codon (64-79) | P=S |
| 2302 | 22 | 3'-Untranslated (2114-2133) | P=S |
| 2303 | 23 | 3'-Untranslated (2039-2058) | P=S |
| 2304 | 24 | 3'-Untranslated (1895-1914) | P=S |
| 2305 | 25 | 3'-Untranslated (1935-1954) | P=S |
| 2307 | 26 | 3'-Untranslated (1976-1995) | P=S |
| 2634 | 1 | AUG-Codon (64-81) | 2'-fluoro A,C & U; P=S |
| 2637 | 15 | 3'-Untranslated (1952-1971) | 2'-fluoro A, C & U; |
| 2691 | 1 | AUG Codon (64-81) | P=O, except last 3 bases, P=S |
| 2710 | 15 | 3'-Untranslated (1952-1971) | 2'-O-methyl; P=O |
| 2711 | 1 | AUG Codon (64-81) | 2'-O-methyl; P=O |
| 2973 | 15 | 3'-Untranslated (1952-1971) | 2'-O-methyl; P=S |
| 2974 | 1 | AUG Codon (64-81) | 2'-O-methyl; P=S |
| 3064 | 27 | 5'-CAP (32-51) | P=S; G & C added as spacer to 3' |
| 3067 | 27 | 5'-CAP (32-51) | P=S |
| 3222 | 27 | 5'-CAP (32-51) | 2'-O-methyl; P=O |
| 3224 | 27 | 5'-CAP (32-51) | 2'-O-methyl; P=S |

Inhibition of ICAM-1 expression on the surface of interleukin-1β-stimulated cells by the oligonucleotides or oligonucleotide analogs was determined by ELISA assay as described in Example 1. The oligonucleotides were tested in two different cell lines. None of the phosphodiester oligonucleotides inhibited ICAM-1 expression. This is probably due to the rapid degradation of phosphodiester oligonucleotides in cells. Of the five phosphorothioate oligonucleotides, the most active was ISIS 1570, which hybridizes to the AUG translation initiation codon.

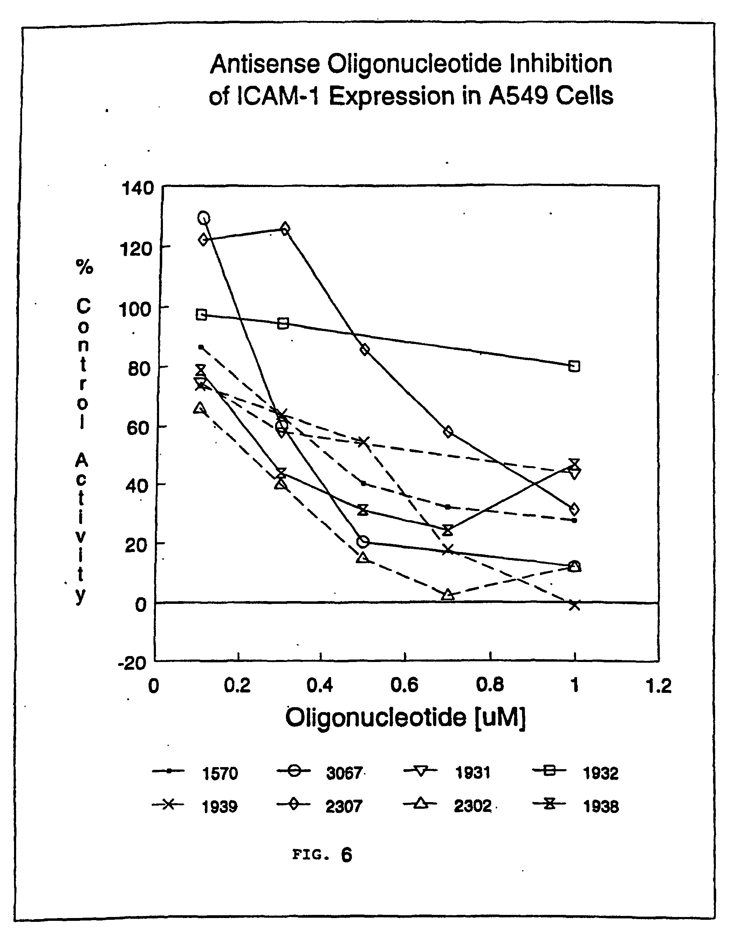

Based on the initial data obtained with the five original targets, twelve more oligonucleotides (ISIS 1930, 1940 and 3067, see Table 1) were designed which would hybridize with the ICAM-1 mRNA. The antisense oligonucleotide (ISIS 3067) which hybridizes to the predicted transcription initiation site (5' cap site) was approximately as active in IL-1β-stimulated cells as the oligonucleotide that hybridizes to the AUG codon (ISIS 1570), as shown in Figure 6. ISIS 1931 and 1932 hybridize 5' and 3', respectively, to the AUG translation initiation codon. All three oligonucleotides that hybridize to the AUG region inhibit ICAM-1 expression, though ISIS 1932 was slightly less active than ISIS 1570 and ISIS 1931. Oligonucleotides which hybridize to the coding region of ICAM-1 mRNA (ISIS 1933, 1934, 1935, 1574 and 1936) exhibited weak activity. Oligonucleotides that hybridize to the translation termination codon (ISIS 1937 and 1938) exhibited moderate activity. Surprisingly, the most active antisense oligonucleotide, ISIS 1939, targeted a specific sequence in the 3'-untranslated region of the ICAM-1 mRNA. The antisense activity demonstrated by ISIS 1939 was not shared by other oligonucleotides (ISIS 1572, 1573, 1940) which hybridize to 3'-untranslated sequences. In fact, ISIS 1940, which targets the polyadenylation signal, failed to inhibit ICAM-1 expression.

Because ISIS 1939 proved unexpectedly to exhibit the greatest antisense activity of the original 16 oligonucleotides tested, other oligonucleotides were designed to hybridize to sequences in the 3'-untranslated region of ICAM-1 mRNA (ISIS 2302, 2303, 2304, 2305, and 2307, as shown in Table 1). ISIS 2307, which hybridizes to a site only five bases 3' to the ISIS 1939 target, was the least active of the series (Figure 6). ISIS 2302, which hybridizes to the ICAM-1 mRNA at a position 143 bases 3' to the ISIS 1939 target, was the most active of the series, with activity comparable to that of ISIS 1939. Examination of the predicted RNA secondary structure of the human ICAM-1 mRNA 3'-untranslated region (according to M. Zuker, *Science,* 244:48-52, 1989) revealed that both ISIS 1939 and ISIS 2302 hybridize to sequences predicted to be in a stable stem-loop structure. Current dogma suggests that regions of RNA secondary structure should be avoided when designing antisense oligonucleotides. Thus, ISIS 1939 and ISIS 2302 would not have been predicted to inhibit ICAM-1 expression.

Several antisense oligonucleotide analogs containing modifications at the 2' position were also analyzed. The 2'-O-methyl phosphorothioate analog of ISIS 1570, ISIS 2974, was approximately threefold less effective than ISIS 1570 in inhibiting ICAM-1 expression in both HUVEC and A549 cells. In contrast, the 2'-O-methyl phosphorothioate analog of ISIS 1939 and ISIS 2973, failed to inhibit ICAM-1 expression in either cell line. Similar results were obtained with 2'-fluoro analogs of ISIS 1570, ISIS 1939 (ISIS 2634 and 2637, respectively).

The control oligonucleotide ISIS 1821 did inhibit. ICAM-1 expression in HUVEC cells with activity comparable to that of ISIS 1934; however, in A549 cells ISIS 1821 was less effective than ISIS 1934, The negative control, ISIS 1821, was found to have a small amount of activity against ICAM expression, probably due in part to its ability to hybridize (12 of 13 base match) to the ICAM-1 mRNA at a position 15 bases 3' to the AUG translation initiation codon.

These studies indicate that the AUG translation initiation codon and specific 3'-untranslated sequences in the ICAM-1 mRNA were the most susceptible to antisense oligonucleotide inhibition of ICAM-1 expression.

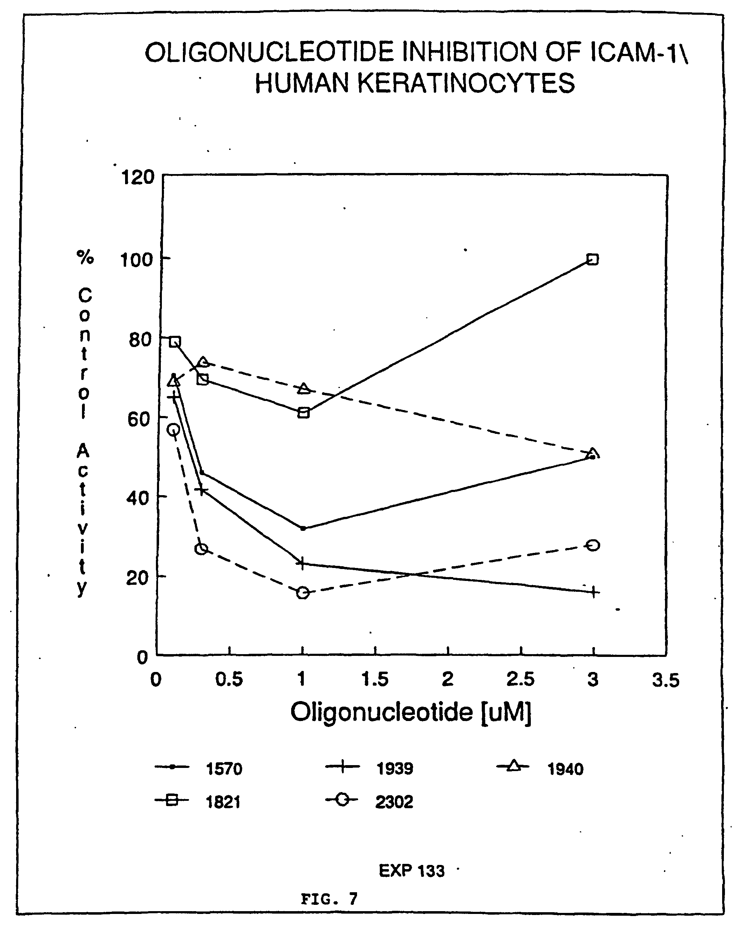

In addition to inhibiting ICAM-1 expression in human umbilical vein cells and the human lung carcinoma cells (A549), ISIS 1570, ISIS 1939 and ISIS 2302 were shown to inhibit ICAM-1 expression in the human epidermal carcinoma A431 cells and in primary human keratinocytes (shown in Figure 7). These data demonstrate that antisense oligonucleotides are capable of inhibiting ICAM-1 expression in several human cell lines. Furthermore, the rank order potency of the oligonucleotides is the same in the four cell lines examined. The fact that ICAM-1 expression could be inhibited in primary human keratinocytes is important because epidermal keratinocytes are an intended target of the antisense nucleotides.

### EXAMPLE 6

### Antisense oligonucleotide inhibition of ICAM-1 expression in cells stimulated with other cytokines:

Two oligonucleotides, ISIS 1570 and ISIS 1939, were tested for their ability to inhibit TNF-α and IFN-γ-induced ICAM-1 expression. Treatment of A549 cells with 1 uM antisense oligonucleotide inhibited IL-1β, TNF-α and IFN-γ-induced ICAM-1 expression in a sequence-specific manner. The antisense oligonucleotides inhibited IL-1β and TNF-α-induced ICAM-1 expression to a similar extent, while IFN-γ-induced ICAM-1 expression was more sensitive to antisense inhibition. The control oligonucleotide, ISIS 1821, did not significantly inhibit IL-1β- or TNF-α-induced ICAM-1 expression and inhibited IFN-γ-induced ICAM-1 expression slightly, as follows:

| **Antisense Oligonucleotide (% Control Expression)** | | | |
|---|---|---|---|
| Cytokine | ISIS 1570 | ISIS 1939 | ISIS 1821 |
| 3 U/ml IL-1β | 56.6 ± 2.9 | 38.1 ± 3.2 | 95 ± 6.6 |
| 1 ng/ml TNF-α | 58.1 ± 0.9 | 37.6 ± 4.1 | 103.5 ± 8.2 |
| 100 U/ml gamma-IFN | 38.9 ± 3.0 | 18.3 ± 7.0 | 83.0 ± 3.5 |

### EXAMPLE 7

### Antisense effects are abolished by sense strand controls:

The antisense oligonucleotide inhibition of ICAM-1 expression by the oligonucleotides ISIS 1570 and ISIS 1939 could be reversed by hybridization of the oligonucleotides with their respective sense strands. The phosphorothioate sense strand for ISIS 1570 (ISIS 1575), when applied alone, slightly enhanced IL-1β-induced ICAM-1 expression. Premixing ISIS 1570 with ISIS 1575 at equal molar concentrations, prior to addition to the cells, blocked the effects of ISIS 1570. The complement to ISIS 1939 (ISIS 2115) enhanced ICAM-1 expression by 46% when added to the cells alone. Prehybridization of ISIS 2115 to ISIS 1939 completely blocked the inhibition of ICAM-1 expression by ISIS 1939.

### EXAMPLE 8

### Measurement of oligonucleotide Tm (dissociation temperature of oligonucleotide from target):

To determine if the potency of the inhibition of ICAM-1 expression by antisense oligonucleotides was due to their affinity for their target sites, thermodynamic measurements were made for each of the oligonucleotides. The antisense oligonucleotides (synthesized as phosphorothioates) were hybridized to their complementary DNA sequences (synthesized as phosphodiesters). Absorbance vs. temperature profiles were measured at 4 µM each strand oligonucleotide in 100 mM Na+, 10 mM phosphate, 0.1 mM EDTA, pH 7.0. Tm's and free energies of duplex formation were obtained from fits of data to a two-state model with linear sloping baselines (Petersheim, M. and D.H. Turner (1983) *Biochemistry,* 22: 256-263). Results are averages of at least three experiments.

When the antisense oligonucleotides were hybridized to their complementary DNA sequences (synthesized as phosphodiesters), all of the antisense oligonucleotides with the exception of ISIS 1940 exhibited a Tm of at least 50°C. All the oligonucleotides should therefore be capable of hybridizing to the target ICAM-1 mRNA if the target sequences were exposed. Surprisingly, the potency of the antisense oligonucleotide did not correlate directly with either Tm or ΔG°₃₇. The oligonucleotide with the greatest biological activity, ISIS 1939, exhibited a Tm which was lower than that of the majority of the other oligonucleotides. Thus, hybridization affinity is not sufficient to ensure biological activity.

### EXAMPLE 9

### Effect of oligonucleotide length on antisense inhibition of ICAM-1 expression:

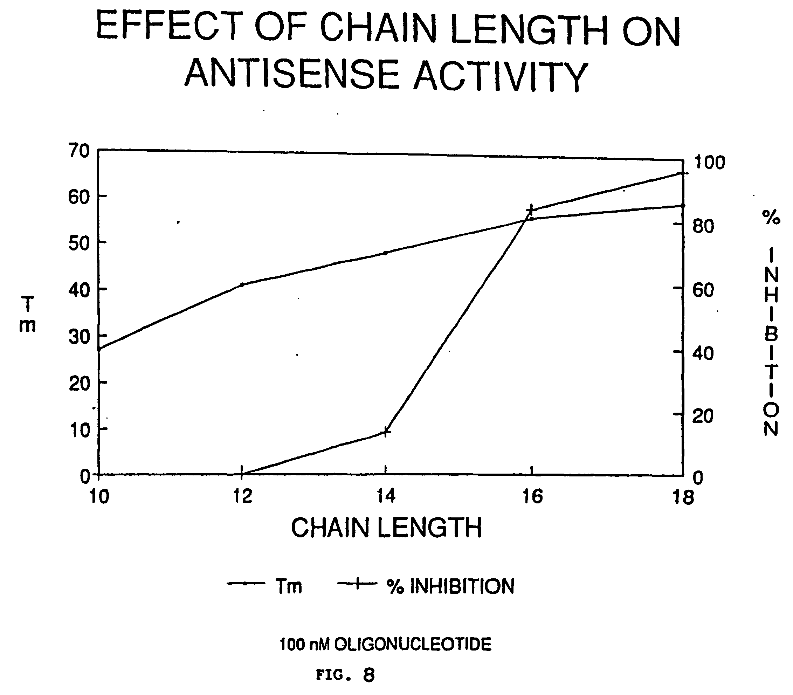

The effect of oligonucleotide length on antisense activity was tested using truncated versions of ISIS 1570 (ISIS 2165, 2173, 2149, 2163 and 2164) and ISIS 1939 (ISIS 2540, 2544, 2545, 2546, 2547 and 2548). In general, antisense activity decreased as the length of the oligonucleotides decreased. Oligonucleotides 16 bases in length exhibited activity slightly less than 18 base oligonucleotides. Oligonucleotides 14 bases in length exhibited significantly less activity, and oligonucleotides 12 or 10 bases in length exhibited only weak activity. Examination of the relationship between oligonucleotide length and Tm and antisense activity reveals that a sharp transition occurs between 14 and 16 bases in length, while Tm increases linearly with length (Figure 8).

### EXAMPLE 10

### Specificity of antisense inhibition of ICAM-1:

The specificity of the antisense oligonucleotides ISIS 1570 and ISIS 1939 for ICAM-1 was evaluated by immunoprecipitation of ³⁵S-labelled proteins. A549 cells were grown to confluence in 25 cm² tissue culture flasks and treated with antisense oligonucleotides as described in Example 4. The cells were stimulated with interleukin-1β for 14 hours, washed with methionine-free DMEM plus 10% dialyzed fetal calf serum, and incubated for 1 hour in methionine-free medium containing 10% dialyzed fetal calf serum, 1 µM oligonucleotide and interleukin-1β as indicated. ³⁵S-Methionine/cysteine mixture (Tran³⁵S-label, purchased from ICN, Costa Mesa, CA) was added to the cells to an activity of 100 µCi/ml and the cells were incubated an additional 2 hours. Cellular proteins were extracted by incubation with 50 mM Tris-HCl pH 8.0, 150 mM NaCl, 1.0% NP-40, 0.5% deoxycholate and 2mM EDTA (0.5 ml per well) at 4°C for 30 minutes. The extracts were clarified by centrifugation at 18,000 x g for 20 minutes. The supernatants were preadsorbed with 200 µl protein G-Sepharose beads (Bethesda Research Labs, Bethesda MD) for 2 hours at 4°C, divided equally and incubated with either 5 µg ICAM-1 monoclonal antibody (purchased from AMAC Inc., Westbrook ME) or HLA-A,B antibody (W6/32, produced by murine hybridoma cells obtained from the American Type Culture Collection, Bethesda, MD) for 15 hours at 4°C. Immune complexes were trapped by incubation with 200 µl of a 50% suspension of protein G-Sepharose (v/v) for 2 hours at 4°C, washed 5 times with lysis buffer and resolved on an SDS-polyacrylamide gel. Proteins were detected by autoradiography.

Treatment of A549 cells with 5 units/ml of interleukin-1β was shown to result in the synthesis of a 95-100 kDa protein migrating as a doublet which was immunoprecipitated with the monoclonal antibody to ICAM-1. The appearance as a doublet is believed to be due to differently glycosylated forms of ICAM-1. Pretreatment of the cells with the antisense oligonucleotide ISIS 1570 at a concentration of 1 µM decreased the synthesis of ICAM-1 by approximately 50%, while 1 µM ISIS 1939 decreased ICAM-1 synthesis to near background. Antisense oligonucleotide ISIS 1940, inactive in the ICAM-1 ELISA assay (Examples 1 and 5) did not significantly reduce ICAM-1 synthesis. None of the antisense oligonucleotides hybridizable with ICAM-1 targets had a demonstrable effect on HLA-A, B synthesis, demonstrating the specificity of the oligonucleotides for ICAM-1. Furthermore, the proteins which nonspecifically precipitated with the ICAM-1 antibody and protein G-Sepharose were not significantly affected by treatment with the antisense oligonucleotides.

### EXAMPLE 11

### Screening of additional antisense oligonucleotides and analogs for activity against ICAM-1 by cell adhesion assay:

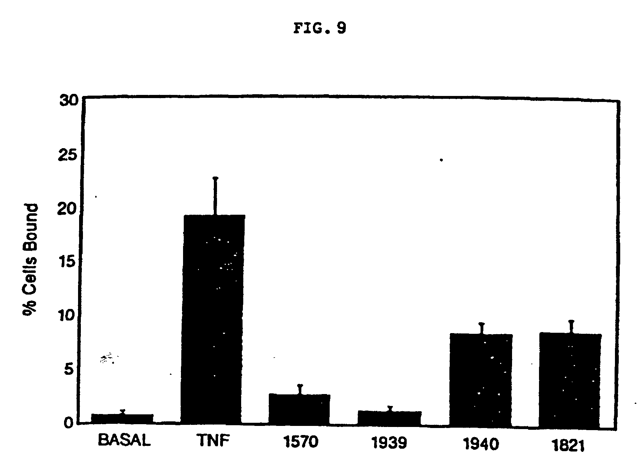

Human umbilical vein endothelial (HUVEC) cells were grown and treated with oligonucleotides as in Example 4. Cells were treated with either antisense oligonucleotide ISIS 1939, the inactive antisense oligonucleotide ISIS 1940, or the control oligonucleotide ISIS 1821 for 4 hours, then stimulated with TNF-α for 20 hours. Basal HUVEC minimally bound HL-60 cells, while TNF-stimulated HUVEC bound 19% of the total cells added. Pretreatment of the HUVEC monolayer with 0.3 µM ISIS 1939 reduced the adherence of HL-60 cells to basal levels, as shown in Figure 9 . The control oligonucleotide, ISIS 1821 and the inactive oligonucleotide ISIS 1940 reduced the percentage of cells adhering from 19% to 9%. These data indicate that antisense oligonucleotides targeting ICAM-1 may specifically decrease adherence of a leukocyte-like cell line (HL-60) to TNF-α-treated HUVEC.

## Claims

1. An oligonucleotide, or oligonucleotide analog, capable of inhibiting the function of nucleic acids encoding intercellular adhesion proteins, which comprises any one of the SEQ IDs 1, 3, 7, 8, 13, 14, 15, 21, 22, 23, 24, 25, 26 and 27.

2. An oligonucleotide, or oligonucleotide analog, according to claim 1, consisting of SEQ ID:22.

3. An oligonucleotide, or oligonucleotide analog, according to any one of the preceding claims comprising 2'-O-methyl, 2'-fluoro, phosphorothioate, 2'-O-memyl-phosphorothioate, or 2'-fluoro-phosphorothioate substituted subunits.

4. An oligonucleotide, or oligonucleotide analog, according to any one of the preceding claims in a pharmaceutically acceptable carrier.

5. An oligonucleotide, or oligonucleotide analog, according to any one of the preceding claims for use in therapy.

6. The use of an oligonucleotide, or oligonucleotide analog, according to any one of claims 1 to 4 in the preparation of a composition for modulating the synthesis or metabolism of an intercellular adhesion molecule.

## Patentansprüche

1. Oligonukleotid oder Oligonukleotidanalogon, befähigt die Funktion von Nukleinsäuren, die interzelluläre Adhäsionsproteine kodieren, zu hemmen, umfassend irgendeine der SEQ-ID-Nrn. 1, 3, 7, 8, 13, 14, 15, 21, 22, 23, 24, 25, 26 und 27.

2. Oligonukleotid oder Oligonukleotidanalogon nach Anspruch 1, welches aus SEQ-ID-Nr. 22 besteht.

3. Oligonukleotid oder Oligonukleotidanalogon nach einem der vorhergehenden Ansprüche, welches 2'-O-Methyl-, 2'-Fluor-, Phosphorthioat-, 2'-O-Methyl-Phosphorthioat- oder 2'-Fluor-Phosphorthioat-substituierte Untereinheiten umfaßt.

4. Oligonukleotid oder Oligonukleotidanalogon nach einem der vorhergehenden Ansprüche in einem pharmazeutisch verträglichen Träger.

5. Oligonukleotid oder Oligonukleotidanalogon nach einem der vorhergehenden Ansprüche zur therapeutischen Verwendung.

6. Verwendung eines Oligonukleotids oder Oligonukleotidanalogon nach einem der Ansprüche 1 bis 4 zur Herstellung einer Verbindung zur Modulation der Synthese oder des Metabolismus eines interzellulären Adhäsionsmoleküls.

## Revendications

1. Un oligonucléotide, ou un analogue d'un oligonucléotide, capable d'inhiber le fonctionnement d'acides nucléiques codant pour des protéines impliquées dans l'adhérence intercellulaire, qui comporte une séquence quelconque parmi SEQ ID 1, 3, 7, 8, 13, 14, 15, 21, 22, 23, 24, 25, 26 et 27.

2. Un oligonucléotide, ou un analogue d'un oligonucléotide, selon la revendication 1, consistant en la séquence SEQ ID:22.

3. Un oligonucléotide, ou un analogue d'un oligonucléotide, selon l'une quelconque des revendications précédentes comportant des sous-unités substituées par du 2'-O-méthyl, 2'-fluoro phosphorothioate, du 2'-O-méthyl-phosphorothioate ou du 2'-fluoro-phosphorothioate.

4. Un oligonucléotide, ou un analogue d'un oligonucléotide, selon l'une quelconque des revendications précédentes, dans un vecteur acceptable pharmaceutiquement.

5. Un oligonucléotide, ou un analogue d'un oligonucléotide, selon l'une quelconque des revendications précédentes, destiné à une utilisation thérapeutique.

6. L'utilisation d'un oligonucléotide, ou d'un analogue d'un oligonucléotide, selon l'une quelconque des revendications 1 à 4 précédentes, dans la préparation d'une composition destinée à moduler la synthèse ou le métabolisme d'une molécule impliquée dans l'adhérence intercellulaire.


### SEQUENCE LISTING

(1) GENERAL INFORMATION:
   (i) APPLICANT: Bennett et al.
   (ii) TITLE OF INVENTION: Oligonucleotide Modulation of Cell Adhesion
   (iii) NUMBER OF SEQUENCES: 39
   (iv) CORRESPONDENCE ADDRESS:
      (A) ADDRESSEE: Woodcock Washburn Kurtz Mackiewicz & Norris
      (B) STREET: One Liberty Place - 46th Floor
      (C) CITY: Philadelphia
      (D) STATE: PA
      (E) COUNTRY: USA
      (F) ZIP: 19103
   (v) COMPUTER READABLE FORM:
      (A) MEDIUM TYPE: DISKETTE, 3.5 INCH, 1.44 Mb STORAGE
      (B) COMPUTER: IBM PS/2
      (C) OPERATING SYSTEM: PC-DOS
      (D) SOFTWARE: WORDPERFECT 5.0
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: 567,286
      (B) FILING DATE: August 14, 1990
   (viii) ATTORNEY/AGENT INFORMATION:
      (A) NAME: Jane Massey Licata
      (B) REGISTRATION NUMBER: 32,257
      (C) REFERENCE/DOCKET NUMBER: ISIS-0334
   (ix) TELECOMMUNICATION INFORMATION:
      (A) TELEPHONE: (215) 568-3100
      (B) TELEFAX: (215) 568-3439
(2) INFORMATION FOR SEQ ID NO:1:
   (i) SEQUENCE CHARACTERISTICS:
      (A) LENGTH: 18
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 1:
   TGGGAGCCAT AGCGAGGC 18
(2) INFORMATION FOR SEQ ID NO: 2:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 2:
   GAGGAGCTCA GCGTCGACTG 20
(2) INFORMATION FOR SEQ ID NO: 3:
   (i) SEQUENCE CHARACTERISTICS:
      (A) LENGTH: 21
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 3:
   GACACTCAAT AAATAGCTGG T 21
(2) INFORMATION FOR SEQ ID NO: 4:
   (i) SEQUENCE CHARACTERISTICS:
      (A) LENGTH: 18
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 4:
   GAGGCTGAGG TGGGAGGA 18
(2) INFORMATION FOR SEQ ID NO: 5:
   (i) SEQUENCE CHARACTERISTICS:
      (A) LENGTH: 18
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 5:
   CGATGGGCAG TGGGAAAG 18
(2) INFORMATION FOR SEQ ID NO: 6:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 6:
   GGGCGCGTGA TCCTTATAGC 20
(2) INFORMATION FOR SEQ ID NO: 7:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 7:
   CATAGCGAGG CTGAGGTTGC 20
(2) INFORMATION FOR SEQ ID NO: 8:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 8:
   CGGGGGCTGC TGGGAGCCAT 20
(2) INFORMATION FOR SEQ ID NO: 9:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 9:
   AGAGCCCCGA GCAGGACCAG 20
(2) INFORMATION FOR SEQ ID NO: 10:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 10:
   TGCCCATCAG GGCAGTTTGA 20
(2) INFORMATION FOR SEQ ID NO: 11:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 11:
   GGTCACACTG ACTGAGGCCT 20
(2) INFORMATION FOR SEQ ID NO: 12:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 12:
   CTCGCGGGTG ACCTCCCCTT   20
(2) INFORMATION FOR SEQ ID NO: 13:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 13:
   TCAGGGAGGC GTGGCTTGTG 20
(2) INFORMATION FOR SEQ ID NO: 14:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 14:
(2) INFORMATION FOR SEQ ID NO: 15:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 15:
   CCCCCACCAC TTCCCCTCTC 20
(2) INFORMATION FOR SEQ ID NO: 16:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 16:
   TTGAGAAAGC TTTATTAACT 20
(2) INFORMATION FOR SEQ ID NO: 17:
   (i) SEQUENCE CHARACTERISTICS:
      (A) LENGTH: 14
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 17:
   AGCCATAGCG AGGC 14
(2) INFORMATION FOR SEQ ID NO: 18:
   (i) SEQUENCE CHARACTERISTICS:
      (A) LENGTH: 12
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 18:
   CCATAGCGAG GC 12
(2) INFORMATION FOR SEQ ID NO: 19:
   (i) SEQUENCE CHARACTERISTICS:
      (A) LENGTH: 10
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 19:
   ATAGCGAGGC 10
(2) INFORMATION FOR SEQ ID NO: 20:
   (i) SEQUENCE CHARACTERISTICS:
      (A) LENGTH: 16
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 20:
   TGGGAGCCAT AGCGAG 16
(2) INFORMATION FOR SEQ ID NO: 21:
   (i) SEQUENCE CHARACTERISTICS:
      (A) LENGTH: 16
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 21:
   GGAGCCATAG CGAGGC 16
(2) INFORMATION FOR SEQ ID NO: 22:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 22:
   GCCCAAGCTG GCATCCGTCA 20
(2) INFORMATION FOR SEQ ID NO: 23:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 23:
   TCTGTAAGTC TGTGGGCCTC 20
(2) INFORMATION FOR SEQ ID NO: 24:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 24:
   AGTCTTGCTC CTTCCTCTTG 20
(2) INFORMATION FOR SEQ ID NO: 25:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 25:
   CTCATCAGGC TAGACTTTAA 20
(2) INFORMATION FOR SEQ ID NO: 26:
   (i) SEQUENCE CHARACTERISTICS:
      (A) LENGTH: 20
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 26:
   TGTCCTCATG GTGGGGCTAT 20
(2) INFORMATION FOR SEQ ID NO: 27:
   (i) SEQUENCE CHARACTERISTICS:
      (A) LENGTH: 22
      (B) TYPE: Nucleic Acid
      (C) STRANDEDNESS: Single
      (D) TOPOLOGY: Linear
   (iv) ANTI-SENSE: Yes
   (xi) SEQUENCE DESCRIPTION: SEQ ID NO: 27:
   TCTGAGTAGC AGAGGAGCTC GA 22